# EUROPEAN PATENT APPLICATION

(11) **EP 2 984 951 A1**
(43) Date of publication of application: **17.02.2016**
(21) Application number: 14186106.2
(22) Date of filing: 24.09.2014
(51) Int. Cl.: A24F 47/00, A61M 15/06

(54) **Electronic cigarette**

(30) Priority: 14.08.2014 CN 201410400132; 14.08.2014 CN 201420460710 U
(71) Applicant: Liu, Tuanfang, 343200 Ji'an City Jiangxi (CN)
(72) Inventor: Liu, Tuanfang, 343200 Ji'an City Jiangxi (CN)
(74) Representative: Hryszkiewicz, Danuta

(57) **Abstract**

An electronic cigarette, including a cigarette holder (1), an atomization rod (3), a transparent tube (5), an oil-guiding cotton (8), a heating wire (9), a copper assembly (10, 11), and a joint (13). The cigarette holder is welded to the transparent tube. The oil-guiding cotton encircles the heating wire. The heating wire has a positive end and a negative end which are separated by an insulating silicone ring (12). The oil-guiding cotton and the heating wire are disposed in the copper assembly. The copper assembly comprises a thread ring (11) and a sealing ring (10). The sealing ring is disposed at one end of the thread ring. The copper assembly is disposed inside the transparent tube. One end of the heating wire is connected to the thread ring, and the other end of the heating wire is connected to the joint. The cigarette holder and the heating wire are connected via the atomization rod.

## Description

The invention relates to an electronic cigarette.

It is well-known that smoking is harmful to health, but there are still over a billion smokers in the world, and the number of smokers is only growing. Many governments have responded to this trend by prohibiting smoking in public places. As a result, nicotine-containing cigarette substitutes, such as patches, mouthwash, gums, drinks, etc., have proliferated. These substitutes, however, make it difficult to achieve a nicotine rush and deprive users of the desired puffing action.

In view of the above-described problems, it is one objective of the invention to provide an electronic cigarette that is convenient for use and safe to the environment. When consuming the electronic cigarette, an enjoyable experience resulting from the smoking actions such as inhaling, exhaling, and puffing can be achieved.

To achieve the above objective, in accordance with one embodiment of the invention, there is provided an electronic cigarette, comprising a cigarette holder, an atomization rod, a transparent tube, an oil-guiding cotton, a heating wire, a copper assembly, and a joint. The cigarette holder is welded to the transparent tube in the presence of ultrasonic waves. The oil-guiding cotton encircles the heating wire. The heating wire comprises a positive end and a negative end which are separated by an insulating silicone ring. The oil-guiding cotton and the heating wire are disposed in the copper assembly. The copper assembly comprises a thread ring and a sealing ring. The sealing ring is disposed at one end of the thread ring. The copper assembly comprising the thread ring and the sealing ring is disposed inside the transparent tube. One end of the heating wire is connected to the thread ring, and the other end of the heating wire is connected to the joint. The cigarette holder and the heating wire are connected via the atomization rod.

In a class of this embodiment, the transparent tube comprises an inner hexagonal hole; and the thread ring comprises an outer hexagonal ridge corresponding to the inner hexagonal hole.

In a class of this embodiment, the transparent tube comprises an inner square hole; and the thread ring comprises an outer square ridge corresponding to the inner square hole.

In a class of this embodiment, the transparent tube is made of transparent polycarbonate material.

In a class of this embodiment, the transparent tube comprises an oil filling opening, and a seal plug is connected to the oil filling opening.

In a class of this embodiment, a first sealing ring and a second sealing ring are disposed at two ends of the atomization rod, respectively; the second sealing ring is connected to the sealing ring of the copper assembly; the thread ring is hollow, one end thereof is sleeved by the sealing ring, and the joint is inserted into the copper assembly from the other end of the thread ring; and the insulating silicone ring is disposed between the joint and the thread ring.

In a class of this embodiment, the heating wire is vertically disposed.

Advantages of the invention are summarized as follows:
1. The transparent tube is made of transparent polycarbonate material, so it is easily adapted to the temperature change, not easy to crack, and has high impact strength;
2. The connection of the cigarette holder and the transparent tube using ultrasonic welding is reliable;
3. The connection of the thread ring and the transparent tube is achieved using a square structure, which is firm and beneficial to the load of the battery; and
4. The smoke oil is added via the oil filling opening without the need to disassemble the electronic cigarette, which is convenient for use.

The invention is described hereinbelow with reference to accompanying drawings, in which:
FIG. 1 is an exploded view of an electronic cigarette of the invention; and
FIG. 2 is a schematic diagram of an electronic cigarette of the invention.

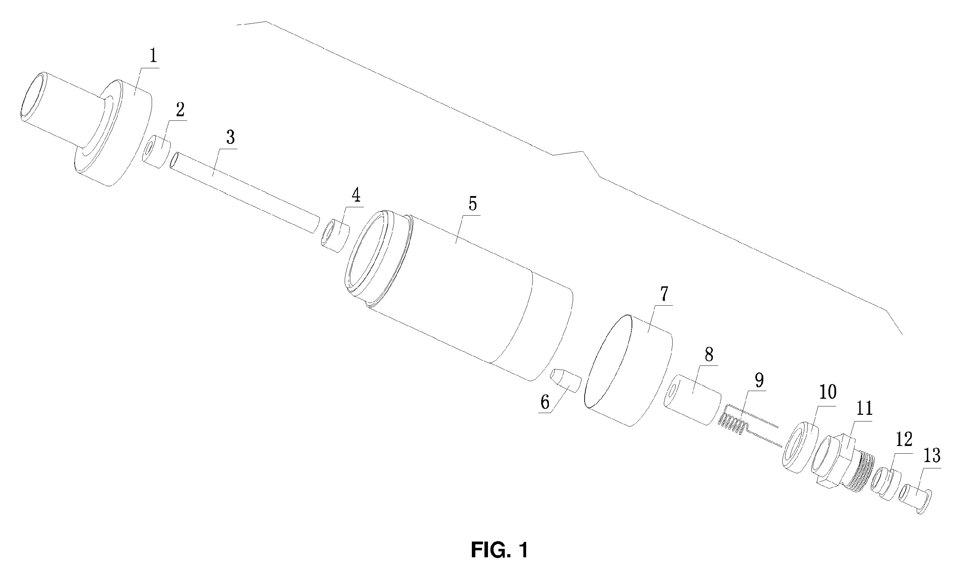

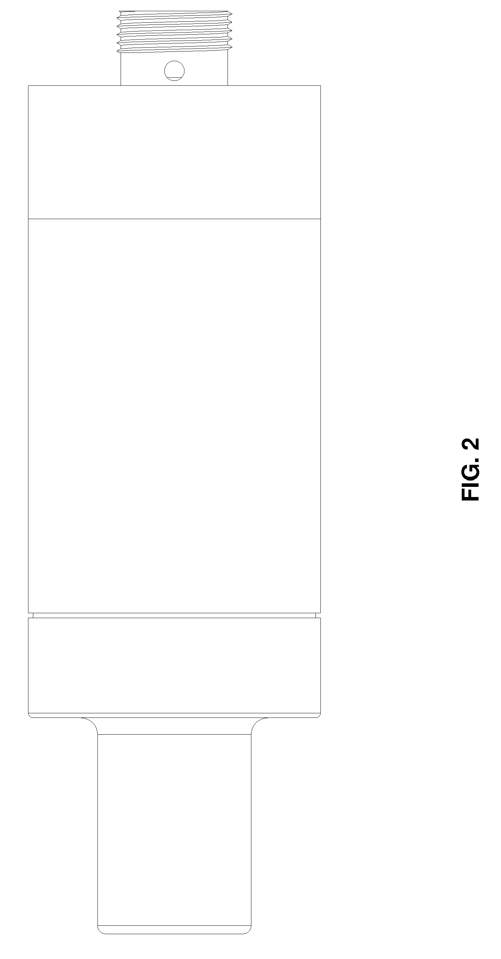

As shown in FIGS. 1 and 2, an electronic cigarette comprises a cigarette holder 1, an atomization rod 3, a transparent tube 5, an oil-guiding cotton 8, a heating wire 9, and a joint 13. The cigarette holder 1 is welded to the transparent tube 5 in the presence of ultrasonic waves. The oil-guiding cotton 8 encircles the heating wire 9. The heating wire 9 comprises a positive end and a negative end which are separated by an insulating silicone ring. The oil-guiding cotton 8 and the heating wire 9 are disposed in a copper assembly. The copper assembly comprises a thread ring 11 and a sealing ring 10. The sealing ring is disposed at one end of the thread ring 11. The copper assembly comprising the thread ring 11 and the sealing ring 10 is disposed inside the transparent tube 5. One end of the heating wire 9 is connected to the thread ring 11, and the other end of the heating wire 9 is connected to the joint 13. The cigarette holder 1 and the heating wire 9 are connected via the atomization rod 3.

Preferably, the transparent tube 5 comprises an inner hexagonal hole. The thread ring 11 comprises an outer hexagonal ridge corresponding to the inner hexagonal hole. When assembling, the hexagonal structures of the transparent tube 5 and the thread ring 11 match with one another and are pressed, whereby limiting the relative rotation between the transparent tube 5 and the thread ring 11.

Preferably, the transparent tube 5 comprises an inner square hole. The thread ring 11 comprises an outer square ridge corresponding to the inner square hole. When assembling, the square structures of the transparent tube 5 and the thread ring 11 match with one another and are pressed, whereby limiting the relative rotation between the transparent tube 5 and the thread ring 11.

Preferably, the transparent tube 5 is made of transparent polycarbonate material.

Preferably, the transparent tube 5 comprises an oil filling opening. The oil filling opening cooperates with a seal plug 6. When the atomizer has been assembled, the smoke oil is added via the oil filling opening, and then the seal plug 6 is plugged in the oil filling opening.

Preferably, a first sealing ring 2 and a second sealing ring 4 are disposed at two ends of the atomization rod 3, respectively. The second sealing ring is connected to the sealing ring 10 of the copper assembly. The thread ring 11 is hollow, one end thereof is sleeved by the sealing ring 10, and the joint is inserted into the copper assembly from the other end of the thread ring. The insulating silicone ring 12 is disposed between the joint 13 and the thread ring 11.

Preferably, a label 7 is attached to the transparent tube 5.

Preferably, the heating wire 9 is vertically disposed in the electronic cigarette, as shown in FIG. 1. Thus, the contact area of the heating wire and the oil-guiding cotton is enlarged, the smoke volume is increased, and the service life of the heating wire is prolonged.

The connection of the cigarette holder 1 and the transparent tube 5 using ultrasonic welding is reliable and oil-tight. The heating wire is vertically disposed, the oil-guiding cotton encircles the heating wire, and the two ends of the heating wire are separated by the insulating silicone ring.

The thread ring and the transparent tube both employ a hexagonal structure to achieve the connection thereof, which can prevent the relative rotation.

Advantages according to embodiments of the invention are summarized as follows.
1. The transparent tube is made of transparent polycarbonate material, so it is easily adapted to the temperature change, not easy to crack, and has high impact strength;
2. The connection of the cigarette holder and the transparent tube using ultrasonic welding is reliable;
3. The connection of the thread ring and the transparent tube is achieved using a square structure, which is firm and beneficial to the load of the battery;
4. The smoke oil is added via the oil filling opening without the need to disassemble the electronic cigarette, which is convenient for use.

While particular embodiments of the invention have been shown and described, it will be obvious to those skilled in the art that changes and modifications may be made without departing from the invention in its broader aspects, and therefore, the aim in the appended claims is to cover all such changes and modifications as fall within the true spirit and scope of the invention.

## Claims

1. An electronic cigarette, comprising:
a) a cigarette holder;
b) an atomization rod;
c) a transparent tube;
d) an oil-guiding cotton;
e) a heating wire;
f) a copper assembly; and
g) a joint;
**characterized in that**
the cigarette holder is welded to the transparent tube;
the oil-guiding cotton encircles the heating wire;
the heating wire comprises a positive end and a negative end which are separated by an insulating silicone ring;
the oil-guiding cotton and the heating wire are disposed in the copper assembly;
the copper assembly comprises a thread ring and a sealing ring; the sealing ring is disposed at one end of the thread ring;
the copper assembly comprising the thread ring and the sealing ring is disposed inside the transparent tube;
one end of the heating wire is connected to the thread ring, and the other end of the heating wire is connected to the joint; and
the cigarette holder and the heating wire are connected via the atomization rod.

2. The electronic cigarette of claim 1, **characterized in that** the transparent tube comprises an inner hexagonal hole; and the thread ring comprises an outer hexagonal ridge corresponding to the inner hexagonal hole.

3. The electronic cigarette of claim 1, **characterized in that** the transparent tube comprises an inner square hole; and the thread ring comprises an outer square ridge corresponding to the inner square hole.

4. The electronic cigarette of claim 1, **characterized in that** the transparent tube is made of transparent polycarbonate material.

5. The electronic cigarette of claim 1, **characterized in that** the transparent tube comprises an oil filling opening, and a seal plug is connected to the oil filling opening.

6. The electronic cigarette of claim 1, **characterized in that** a first sealing ring and a second sealing ring are disposed at two ends of the atomization rod, respectively; the second sealing ring is connected to the sealing ring of the copper assembly; the thread ring is hollow, one end thereof is sleeved by the sealing ring, and the joint is inserted into the copper assembly from the other end of the thread ring; and the insulating silicone ring is disposed between the joint and the thread ring.

7. The electronic cigarette of claim 1, **characterized in that** the heating wire is vertically disposed.
